# EUROPÄISCHE PATENTANMELDUNG

(11) **EP 4 699 514 A1**
(43) Veröffentlichungstag der Anmeldung: **25.02.2026**
(21) Anmeldenummer: 25195844.3
(22) Anmeldetag: 14.08.2025
(51) Int. Cl.: A61B 1/00, A61B 1/12, G01N 21/94

(54) **VORRICHTUNG UND VERFAHREN ZUM BESTIMMEN EINES ZUSTANDS EINES OPTISCHEN SYSTEMS FÜR EIN ENDOSKOP**

(30) Priorität: 19.08.2024 DE 102024123656
(71) Anmelder: Karl Storz SE & Co. KG, 78532 Tuttlingen (DE)
(72) Erfinder: BRÜDERLE, Klaus, 78532 Tuttlingen (DE)

(57) **Zusammenfassung**

Die Erfindung stellt eine Vorrichtung (100) sowie ein System zum Bestimmen eines Zustands eines optischen Systems (125) für ein Endoskop (120) bereit. Die Vorrichtung umfasst:
eine Eingangsschnittstelle (110), welche dazu eingerichtet ist, mittels des optischen Systems (125) erfasste Bilddaten (71) zu erhalten;
einer Recheneinrichtung (150), welche dazu eingerichtet ist, zumindest ein Auswertemodul (152) zu implementieren, welches dazu eingerichtet ist, einen gesamten optischen Fluss der erhaltenen Bilddaten (71) zu bestimmen, darauf basierend mindestens einen Bildflussvektor (22, 25) zu identifizieren, und, falls mindestens zwei verschiedene Bildflussvektoren (22, 25) identifiziert wurden, basierend darauf mindestens einen Zustand des optischen Systems (125) zu bestimmen; und
einer Ausgabeschnittstelle (190), welche dazu eingerichtet ist, ein Ausgabesignal (79) zu erzeugen, welches eine Information über den bestimmten mindestens einen Zustand des optischen Systems (125) aufweist.

## Beschreibung

### Technisches Gebiet der Erfindung

Die vorliegende Erfindung betrifft ein System sowie ein Verfahren zum Bestimmen eines Zustands eines optischen Systems für ein Endoskop. Bei dem Zustand kann es sich insbesondere um eine Beeinträchtigung des optischen Systems handeln, beispielsweise um eine Beschädigung oder eine Verschmutzung des optischen Systems.

### Hintergrund der Erfindung

Endoskope sind aus dem chirurgischen Instrumentarium nicht mehr wegzudenken. Sie ermöglichen es einem Operateur, detailgenaue und akkurate Kenntnis einer Operationsstelle zu erlangen, ohne dass diese vollständig geöffnet werden muss. Ein einwandfreier Zustand des Endoskops, insbesondere des optischen Systems des Endoskops, ist somit von großer Bedeutung, da Beeinträchtigungen des optischen Systems die Arbeit des Operateurs erschweren können.

In manchen Fällen ist es für den Operateur nämlich nicht ersichtlich, ob ein ihm dargestelltes graphisches Bild einwandfrei ist, oder ob es teilweise auf eine Beeinträchtigung des optischen Systems des Endoskops zurückgeht. Beispielsweise könnte sich das optische System in einem verschmutzten oder beschädigten Zustand befinden.

### Zusammenfassung der Erfindung

Es ist daher eine Aufgabe der vorliegenden Erfindung, eine Vorrichtung sowie ein Verfahren bereitzustellen, welche die zuverlässige Bestimmung eines Zustands eines optischen Systems eines Endoskops ermöglicht.

Diese Aufgabe wird durch die Gegenstände der unabhängigen Ansprüche der vorliegenden Erfindung gelöst.

Gemäß einem ersten Aspekt wird eine Vorrichtung zum Bestimmen eines Zustands eines optischen Systems für ein Endoskop bereitgestellt, umfassend:
eine Eingangsschnittstelle, welche dazu eingerichtet ist, mittels des optischen Systems erfasste Bilddaten zu erhalten;
einer Recheneinrichtung, welche dazu eingerichtet ist, zumindest ein Auswertemodul zu implementieren, welches dazu eingerichtet ist, einen gesamten optischen Fluss (oder: optischen Hauptfluss) der erhaltenen Bilddaten zu bestimmen, darauf basierend mindestens einen Bildflussvektor zu identifizieren, und, falls mindestens zwei verschiedene Bildflussvektoren identifiziert wurden (optional: und außerdem ein globaler Bildfluss festgestellt wurde), basierend darauf mindestens einen Zustand des optischen Systems zu bestimmen; und
einer Ausgabeschnittstelle, welche dazu eingerichtet ist, ein Ausgabesignal zu erzeugen, welches eine Information über den bestimmten mindestens einen Zustand des optischen Systems aufweist.
Wenn hierin von optischen Bildflussvektoren die Rede ist, so sollen darunter insbesondere Hauptvektoren (oder: dominante optische Bildflussvektoren) verstanden werden.

Eine grundlegende Idee der vorliegenden Erfindung besteht darin, dass der optische Fluss von Bilddaten eines Endoskops ausgewertet wird, um den aktuellen Zustand des Endoskops, insbesondere des optischen Systems des Endoskops, zu bestimmen.

Vorteilhaft ist das Auswertemodul außerdem dazu eingerichtet, zu prüfen, ob ein globaler Bildfluss vorliegt, d.h., ob in einer bestimmten Folge (oder: Sequenz) von Bilddaten Bildflussvektoren darauf beruhen, dass eine Bewegung des optischen Systems (als Ganzes) vorlag, statt beispielsweise eine Bewegung von einzelnen Objekten oder Strukturen innerhalb eines von dem optischen System erfassten Bildes. Hierzu kann beispielsweise durch das Auswertemodul ermittelt werden, wie viele nicht-verschwindende (d.h., vom Nullvektor verschiedene) Bildflussvektoren (insbesondere Hauptvektoren, oder: dominante optische Bildflussvektoren) vorliegen, und erst ab einem bestimmen Schwellwert von Y% von Pixeln (oder Bereichen der Bilddaten) mit einem nicht-verschwindenden Bildflussvektor gefolgert werden, dass ein globaler Bildfluss vorliegt. Bei dem Bestimmen des mindestens einen Zustands kann vorgesehen sein, dass nur in solchen Bilddaten auf einen beeinträchtigten Zustand des optischen Systems geschlossen werden kann, in welchen zuvor ein globaler Bildfluss festgestellt wurde. Der Schwellwert Y% zum Erkennen eines globalen Bildflusses kann beispielsweise 40%, 50%, 60%, 70%, 80%, 90%, 95%, 98% oder dergleichen betragen.

Der Schwellwert Y% kann fix sein, oder kann variabel gestaltet sein. Ein variabler Schwellwert Y% kann insbesondere basierend auf mindestens einem zuvor bestimmten Zustand angepasst werden. Wurde beispielsweise in der Vergangenheit bereits festgestellt, dass ein bestimmtes optisches System eine Beeinträchtigung derart aufweist, dass nur noch 90% der Pixel das tatsächliche Bild erfassen, die restlichen aber blind sind, kann der Schwellwert Y% entsprechend gesenkt werden, beispielsweise skaliert mit der Anzahl der unbeeinträchtigten Pixel. Ist beispielsweise als Anfangs-Schwellwert Y%=80% vorgesehen, und sind nur noch 90% der Pixel unbeeinträchtigt oder zuverlässig, kann ein neuer, angepasster Schwellwert auf 90%*80%= 72% festgelegt werden.

Das optische System kann insbesondere ein Objektiv und/oder eine Vielzahl von optischen Leitern (z.B. Glasfasern) umfassen, welche ein durch das Objektiv optisch erfasstes Bild in Lichtsignalen an eine Verarbeitungseinrichtung, beispielsweise die Recheneinrichtung der erfindungsgemäßen Vorrichtung, weiterleiten. Das Objektiv kann eine oder mehrere Linsen aufweisen.

Die erfindungsgemäße Vorrichtung kann bevorzugt ein Endoskop oder zumindest ein optisches System für ein Endoskop, dessen Zustand bestimmt werden soll, umfassen.

Obwohl hier, im Vorstehenden und im Folgenden einige Funktionen als von "Einrichtungen", "Schnittstellen" oder "Modulen" ausgeführt beschrieben werden, versteht es sich, dass dies nicht unbedingt bedeutet, dass solche Einrichtungen, Schnittstellen, oder Module als voneinander getrennte Einheiten bereitgestellt werden. In Fällen, in denen eine oder mehrere Einrichtungen, Schnittstellen, oder Module ganz oder teilweise als Software bereitgestellt werden, können die Einrichtungen, Schnittstellen, oder Module durch Programmcodeabschnitte oder Programmcodeschnipsel implementiert werden, die sich voneinander unterscheiden, aber auch miteinander verwoben sein können.

In ähnlicher Weise können in dem Fall, in dem ein oder mehrere Einrichtungen, Schnittstellen, oder Module als Hardware bereitgestellt werden, die Funktionen einer oder mehrerer Einrichtungen, Schnittstellen, oder Module von ein und derselben Hardwarekomponente bereitgestellt werden, oder die Funktionen einer Einrichtung, einer Schnittstelle, oder eines Moduls oder die Funktionen mehrerer Einrichtungen, Schnittstellen, oder Module können auf mehrere Hardwarekomponenten verteilt sein, die nicht notwendigerweise den Einrichtungen, Schnittstellen, oder Modulen eins zu eins entsprechen müssen. Daher ist jede Vorrichtung, jedes System, jedes Verfahren usw., das alle Merkmale und Funktionen aufweist, die einer bestimmten Einrichtung und/oder einer bestimmten Schnittstelle und/oder einem bestimmen Modul zugeschrieben werden, so zu verstehen, dass sie bzw. es die Einrichtung und/oder die Schnittstelle und/oder das Modul ausbildet, umfasst, oder implementiert.

Insbesondere besteht die Möglichkeit, dass alle Einrichtungen, Schnittstellen, oder Module durch Programmcode implementiert werden, der von einer Recheneinrichtung ausgeführt wird.

Die Recheneinrichtung kann als jede Vorrichtung oder jedes Mittel zum Rechnen realisiert werden, insbesondere zum Ausführen einer Software, einer App oder eines Algorithmus. Beispielsweise kann die Recheneinrichtung mindestens einen Prozessor, wie etwa mindestens einen Zentralprozessor, CPU und/oder mindestens einen Grafikprozessor, GPU, und/oder mindestens ein feldprogrammierbares Gate-Array, FPGA, und/oder mindestens einen anwendungsspezifischen integrierten Schaltkreis, ASIC, und/oder eine beliebige Kombination der Vorstehenden umfassen. Die Recheneinrichtung kann ferner einen Arbeitsspeicher aufweisen, der mit dem mindestens einen Prozessor operativ verbunden ist, und/oder einen nichtflüchtigen Speicher, der mit dem mindestens einen Prozessor und/oder dem Arbeitsspeicher operativ verbunden ist. Die Recheneinrichtung kann teilweise und/oder vollständig in einer lokalen Vorrichtung und/oder teilweise und/oder vollständig in einem entfernten System, wie z. B. durch eine Cloud-Computing-Plattform, implementiert sein.

Gemäß einem zweiten Aspekt stellt die vorliegende Erfindung ein computerimplementiertes Verfahren zum Bestimmen eines Zustands eines optischen Systems für ein Endoskop bereit. Das Verfahren umfasst zumindest die Schritte:
Erhalten von Bilddaten, welche durch das optische System erfasst wurden;
Bestimmen eines gesamten optischen Flusses (oder: optischen Hauptflusses) in den erhaltenen Bilddaten;
Identifizieren mindestens eines Bildflussvektors in dem bestimmten gesamten optischen Fluss; und,
falls mindestens zwei verschiedene Bildflussvektoren identifiziert wurden (optional: und falls außerdem ein globaler Bildfluss festgestellt wurde):
Bestimmen, basierend auf den mindestens zwei verschiedenen identifizierten Bildflussvektoren, mindestens eines Zustands des optischen Systems.

Weitere Schritte können beispielsweise das Erfassen von Bilddaten durch das optische System, das Erzeugen eines Ausgabesignals, das Steuern einer Anzeigeeinrichtung mittels eines Steuersignals und dergleichen umfassen.

Gemäß einem dritten Aspekt stellt die Erfindung ein Computerprogrammprodukt bereit, das ausführbaren Programmcode umfasst, welcher dazu eingerichtet ist, wenn er von einer Recheneinrichtung ausgeführt wird, das Verfahren gemäß einer Ausführungsform des zweiten Aspekts der vorliegenden Erfindung durchzuführen.

Gemäß einem vierten Aspekt stellt die Erfindung ein nicht-flüchtiges, computerlesbares Datenspeichermedium bereit, das ausführbaren Programmcode umfasst, welcher dazu eingerichtet ist, wenn er von einer Recheneinrichtung ausgeführt wird, das Verfahren gemäß einer Ausführungsform des zweiten Aspekts der vorliegenden Erfindung durchzuführen.

Das nicht-flüchtige, computerlesbare Datenspeichermedium kann jede Art von Computerspeicher, insbesondere Halbleiterspeicher, wie z.B. einen Festkörperspeicher, umfassen oder aus einem solchen bestehen. Das Datenträger kann auch eine CD, eine DVD, eine Blu-Ray-Disc, einen USB-Speicherstick oder dergleichen umfassen oder daraus bestehen.

Gemäß einem fünften Aspekt stellt die Erfindung einen Datenstrom bereit, der ausführbaren Programmcode umfasst oder zum Erzeugen von ausführbarem Programmcode konfiguriert ist, welcher dazu eingerichtet ist, wenn er von einer Recheneinrichtung ausgeführt wird, dass Verfahren gemäß einer Ausführungsform des zweiten Aspekts der vorliegenden Erfindung durchzuführen.

Gemäß einigen bevorzugten Ausführungsformen, Varianten oder Verfeinerungen von Ausführungsformen ist das Auswertemodul dazu eingerichtet, anhand der mindestens zwei identifizierten verschiedenen Bildflussvektoren mindestens einen der folgenden Zustände des optischen Systems zu bestimmen:
- eine Verschmutzung des optischen Systems;
- eine Beschädigung des optischen Systems;
- eine verbleibende Nutzungsdauer des optischen Systems; und/oder
- einen Funktionalitätsgrad des optischen Systems.

Eine Verschmutzung des optischen Systems kann beispielsweise einen Fleck oder Fussel auf einem Objektiv des optischen Systems umfassen.

Eine Beschädigung des optischen Systems kann eine Beschädigung eines Objektivs des optischen Systems umfassen, beispielsweise einen Kratzer, einen Riss, eine Trübung und/oder dergleichen. Eine Beschädigung des optischen Systems kann auch eine Beschädigung mindestens eines Lichtleiters umfassen, beispielsweise einen Bruch, eine Splitterung, einen Litzendefekt, eine Alterung und/oder dergleichen.

Bei dem Funktionalitätsgrad kann es sich um einen Prozentsatz handeln, zu welchem das optische System funktionsfähig ist, beispielsweise, zu welchem das optische System einen Raumwinkel, den es im idealen, unbeeinträchtigten Zustand vollständig erfassen könnte, noch erfassen kann. Der Funktionalitätsgrad kann auch als Prozentsatz von funktionsfähigen Pixeln einer Gesamtmenge von im idealen, unbeeinträchtigten Zustand verfügbaren Pixeln oder dergleichen angegeben werden.

Bei dem Auswerten der bestimmten Bildflussvektoren zum Identifizieren des mindestens einen Zustands des optischen Systems kann ein Schwellwert vorgesehen sein, insbesondere eine Mindestanzahl von identifizierten verschiedenen Bildflussvektoren, welche überschritten sein muss, damit das Auswertemodul einen Zustand des optischen Systems bestimmt, welcher eine Beeinträchtigung darstellt. Das Auswertemodul kann so eingerichtet sein, dass es, falls in dem optischen Fluss nur ein einziger Bildflussvektor identifiziert wird (d.h., festgestellt wird, dass sich alle Bildpunkt gleich bewegen/bewegt haben), oder eine Anzahl von verschiedenen Bildflussvektoren bis maximal dem vorgesehenen Schwellwert identifiziert wird, auf einen Standard-Zustand (z.B., "keine Beeinträchtigung" oder "Funktionsfähigkeit 100%") des optischen Systems schließt.

Alternativ kann aber auch kein Schwellwert vorgesehen sein (bzw., gleichwertig, ein Schwellwert von "1 Bildflussvektor" vorgesehen sein), sodass das Auswertemodul bereits bei zwei verschiedenen identifizierten Bildflussvektoren auf einen Zustand "Beeinträchtigung liegt vor" des optischen Systems schließt, sowie bevorzugt daraufhin (oder von Anfang an) eine genauere Bestimmung der Beeinträchtigung vornimmt. Es kann auch vorgesehen sein, dass der Schwellwert der Vorrichtung durch einen Benutzer, z.B. über eine Benutzerschnittstelle, einstellbar ist, je nachdem, welche Anwendung geplant ist. Bei einem voraussichtlich sehr statischen Bild könnte der Schwellwert somit niedriger gesetzt werden als bei einem voraussichtlich sehr veränderlichen Bild.

Das Erfassen des jeweiligen Zustands des optischen Systems kann auch eine quantitative Einstufung des Zustands (insbesondere, wenn diese eine Beeinträchtigung darstellt) umfassen. Beispielsweise kann im Falle einer Verschmutzung oder Beschädigung eines Objektivs des optischen Systems ein Prozentsatz erfasst werden, zu welchem das Objektiv von der Verschmutzung oder Beschädigung betroffen ist und/oder ein Prozentsatz, zu welchem ein durch das Objektiv erfasstes Bild von der Verschmutzung oder Beschädigung betroffen ist. Im Falle von beschädigten Lichtleitern kann ein Prozentsatz von Lichtleitern aus der Gesamtheit der Lichtleiter erfasst werden, welcher beschädigt ist, und dergleichen mehr. Das durch die Ausgabeschnittstelle erzeugte Signal kann auch diese quantitative Einstufung, also etwa einen oder mehrere der genannten Prozentsätze, umfassen, insbesondere in digitaler Form.

Das Erfassen des jeweiligen Zustands des optischen Systems wird bevorzugt mehrfach, insbesondere regelmäßig oder zumindest wiederholt, durchgeführt. Insbesondere wenn auch eine quantitative Einstufung des jeweiligen Zustands bestimmt wird, kann auf diese Weise durch das Auswertemodul eine zeitliche Entwicklung dieser quantitativen Einstufung erfasst werden. Auf dieser Basis kann, beispielsweise wenn für einen oder mehreren quantitative Einstufungen ein Schwellwert vorgegeben ist, eine verbleibende Nutzungsdauer des optischen Systems bestimmt werden. Beispielsweise kann vorgesehen sein, dass bei einer Beschädigung von x% oder mehr der Lichtleiter, und/oder von x% oder mehr der Fläche des Objektivs, das optische System als "nicht mehr funktionsfähig" eingestuft werden soll, d.h., dass das Ende der Nutzungsdauer erreicht ist.

Durch das fortlaufende Bestimmen der quantitativen Einstufung mit jeweiligen Zeitstempeln kann somit ein Zeitpunkt des Endes der Nutzungsdauer des optischen Systems extrapoliert werden. Auf dieser Basis kann beispielsweise eine vorausschauende Wartung (engl. "predictive maintenance") durchgeführt werden, sodass etwa x Wochen oder Monate vor dem extrapolierten (oder: prognostizierten) Ende der Nutzungsdauer eine Warnung ausgegeben wird (z.B. über ein Instrumenten-Verwaltungssystem oder eine Anzeigeeinrichtung der Vorrichtung), wonach in x Wochen bzw. Monaten die Nutzungsdauer des optischen Systems endet. Diese Anzeige kann dauerhaft ausgegeben werden und dynamisch aktualisiert werden, etwa wie eine Tankfüllstandsanzeige eines Fahrzeugs.

Das von der Ausgabeschnittstelle erzeugte Ausgabesignal kann eine Information über ein prognostiziertes Ende der Nutzungsdauer und/oder einen empfohlenen Wartungs- oder Austauschzeitpunkt umfassen und durch die erfindungsgemäße Vorrichtung beispielsweise an ein Instrumenten-Verwaltungssystem übermittelt werden. Darauf basierend kann automatisch ein Ersatzteil bestellt werden, die Produktion eines Ersatzteils gesteuert werden, und dergleichen mehr.

Gemäß einigen bevorzugten Ausführungsformen, Varianten oder Verfeinerungen von Ausführungsformen ist die Ausgabeschnittstelle außerdem dazu eingerichtet, eine Handlungsaufforderung an einen Benutzer zum Beheben oder Verbessern des mindestens einen bestimmten Zustands des optischen Systems auszugeben und/oder ein Steuersignal zum automatischen Beheben oder Verbessern des mindestens einen bestimmten Zustands des optischen Systems auszugeben. Die Handlungsaufforderung und/oder das Steuersignal können Teil des Ausgabesignals der Ausgabeschnittstelle sein, oder von diesem verschieden sein.

Die Handlungsaufforderung kann beispielsweise darin bestehen, dass eine graphische Anzeige, welche das aktuell von dem optischen System erfasste Bild anzeigt, zusätzlich den Hinweis "Objektiv reinigen" oder dergleichen anzeigt, oder eine akustische Warnung (in Form eines Warntons oder einer Sprachausgabe) dasselbe anzeigt. Zum Ausgeben der Handlungsaufforderung können durch die Ausgabeschnittstelle entsprechende Steuersignal ausgegeben werden, etwa Videodatensignale, Audiodatensignale und dergleichen. Im Falle einer (zumindest durch den aktuellen Benutzer) nicht behebbaren Beeinträchtigung gemäß dem bestimmten Zustand des optischen Systems kann die Handlungsaufforderung auch eine Aufforderung zur Wartung oder sogar zum Austausch des optischen Systems beinhalten.

Ein Steuersignal zum automatischen Beheben oder Verbessern des mindestens einen Zustands kann beispielsweise darin bestehen, dass im Fall eines beschädigten Lichtleiters softwareseitig das oder die Bildpixel, welche von diesem Lichtleiter übertragen werden müssten, ausgelassen oder ersetzt wird/werden, etwa durch einen Platzhalter oder aber durch eine Interpolation aus den umgebenden Bildpixeln. Das Bestimmen des Zustands des optischen Systems kann umfassen, zu bestimmen, ob ein solches automatisches Beheben oder Verbessern durchzuführen ist (beispielsweise, wenn die Beeinträchtigung ästhetisch störend, aber nicht gravierend ist) oder zu unterlassen ist (beispielsweise, wenn die Beeinträchtigung mehr als x Lichtleiter umfasst, sodass möglicherweise nicht mehr gewährleistet ist, dass die Interpolation keine Objekte in dem erfassten Bild auslöscht).

Gemäß einigen bevorzugten Ausführungsformen, Varianten oder Verfeinerungen von Ausführungsformen ist das Auswertemodul außerdem dazu eingerichtet, eine Geschwindigkeit einer Veränderung mindestens eines bestimmten Zustands des optischen Systems zu bestimmen. Bei der Veränderung wird es sich üblicherweise um eine Verschlechterung des Zustands, d.h., um eine fortschreitende Degradation des optischen Systems, handeln.

Wie im Vorangehenden bereits erläutert wurde, kann das Bestimmen der Geschwindigkeit der Veränderung insbesondere im Zusammenhang mit, oder basierend auf, einer quantitativen Einstufung des bestimmten Zustands erfolgen. Ausgehend von der bestimmten Geschwindigkeit der Veränderung kann beispielsweise dann auch eine Beschleunigung der Veränderung bestimmt werden, um auch eine mögliche rapide Verschlechterung des Zustands erkennen zu können, etwa für die vorausschauende Wartung.

Gemäß einigen bevorzugten Ausführungsformen, Varianten oder Verfeinerungen von Ausführungsformen ist das Auswertemodul dazu eingerichtet, das Bestimmen des optischen Flusses, das Identifizieren des mindestens einen Bildflussvektors und/oder das Bestimmen des mindestens einen Zustands des optischen Systems in einem Normalbetrieb zur Laufzeit des optischen Systems durchzuführen. Der Benutzer muss somit nicht explizit einen Testmodus oder dergleichen durchführen, sondern führt wie gewohnt seine Tätigkeit durch, während das Auswertemodul im Hintergrund agiert. Auf diese Weise ist auch sichergestellt, dass der Zustand des optischen Systems auch mit einer gewünschten Häufigkeit oder Regelmäßigkeit bestimmt wird.

Gemäß einigen bevorzugten Ausführungsformen, Varianten oder Verfeinerungen von Ausführungsformen ist das Auswertemodul dazu eingerichtet, das Bestimmen des optischen Flusses, das Identifizieren des mindestens einen Bildflussvektors und/oder das Bestimmen des mindestens einen Zustands des optischen Systems ausschließlich (oder zusätzlich zu einer regelmäßigen Durchführung im Hintergrund) in einem gesonderten Testbetrieb des optischen Systems durchzuführen. Somit kann sich der Benutzer vor einem Eingriff, oder bei einem Verdacht, vergewissern, dass das optische System in einem einwandfreien Zustand (oder in einem bekannten, leicht beeinträchtigten Zustand) ist. In dem Testbetrieb können dem Benutzer optional durch das Auswertemodul Handlungsanweisungen zum Durchführen des Testbetriebs bereitgestellt werden. Die Handlungsanweisungen können visuell (durch eine Anzeigeeinrichtung) oder akustisch erfolgen, wie im Vorangehenden bereits erläutert wurde. Die Handlungsanweisungen können Anweisungen umfassen wie etwa: "Bitte von links nach rechts schwenken", "Bitte stillhalten", oder dergleichen.

Weitere vorteilhafte Varianten, Optionen, Ausführungsformen und Modifikationen ergeben sich aus den folgenden Figuren und der zugehörigen ausführlichen Beschreibung, sowie aus den Ansprüchen. Es versteht sich jedoch, dass die detaillierte Beschreibung und die spezifischen Beispiele zwar bevorzugte Ausführungsformen der Erfindung angeben, aber nur zur Veranschaulichung angeführt werden, da verschiedene Änderungen und Modifikationen innerhalb des Umfangs der Erfindung für den Fachmann ersichtlich sind.

### Kurzbeschreibung der Figuren

Einzelne Ausführungsformen der vorliegenden Offenbarung werden anhand der folgenden Abbildungen im Detail erläutert werden. Die Bestandteile in den Zeichnungen sind nicht notwendigerweise maßstabsgetreu, sondern dienen dazu, die Grundsätze der vorliegenden Erfindung zu veranschaulichen. Teile in den verschiedenen Abbildungen, die den gleichen Elementen oder Verfahrensschritten entsprechen, wurden in den Abbildungen mit den gleichen Bezugszeichen versehen. Die Nummerierung von Verfahrensschritten dient zunächst nur zu deren Unterscheidung und impliziert nicht zwingend eine entsprechende Reihenfolge, wobei es jedoch eine Variante darstellt, die Schritte in der Reihenfolge ihrer Nummerierung durchzuführen. Mehrere Schritte können auch überlappend oder gleichzeitig durchgeführt werden. Von den Figuren zeigen:
- Fig. 1: ein schematisches Blockdiagramm zum Erläutern einer Vorrichtung gemäß einer Ausführungsform der vorliegenden Erfindung;
- Fig. 2: schematisch ein optisches System für ein Endoskop;
- Fig.3a bis Fig. 3c: schematisch eine Abfolge von Bilddaten, wie sie von dem optischen System aus Fig. 2 aufgenommen werden;
- Fig. 4: schematische Bildflussvektoren basierend auf der zeitlichen Abfolge der Bilddaten aus Fig. 3a bis Fig. 3c;
- Fig. 5: ein schematisches Flussdiagramm zum Erläutern eines Verfahrens gemäß einer weiteren Ausführungsform der vorliegenden Erfindung;
- Fig. 6: ein schematisches Blockdiagramm eines Computerprogrammprodukts gemäß noch einer weiteren Ausführungsform der vorliegenden Erfindung; und
- Fig. 7: ein schematisches Blockdiagramm eines nichtflüchtigen computerlesbaren Datenspeichermediums gemäß noch einer weiteren Ausführungsform der vorliegenden Erfindung.

### Detaillierte Beschreibung der Figuren

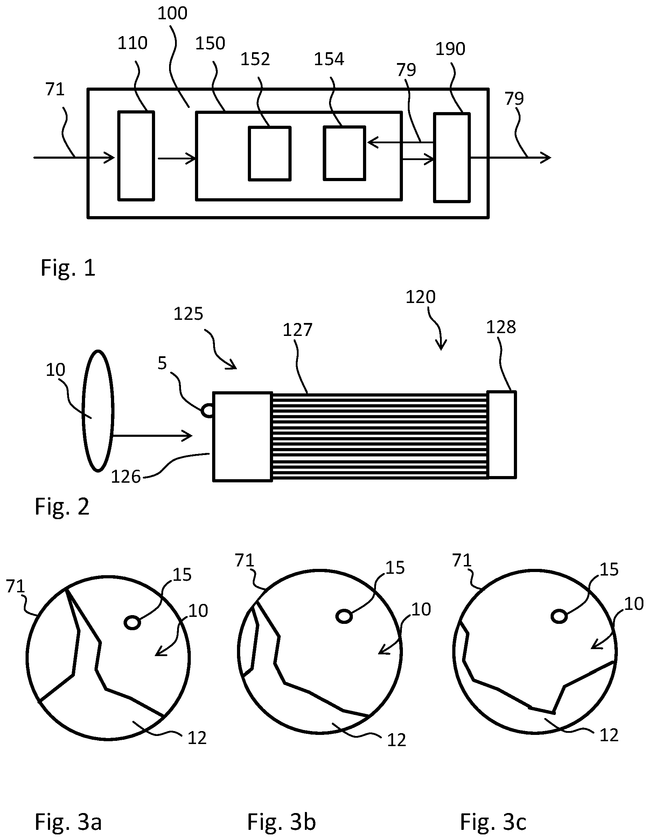

Fig. 1 zeigt ein schematisches Blockdiagramm zum Erläutern einer Vorrichtung 100 gemäß einer Ausführungsform der vorliegenden Erfindung, d.h., einer Vorrichtung 100 zum Bestimmen eines Zustands eines optischen Systems für ein Endoskop.

Fig. 2 zeigt schematisch ein solches optisches System 125 für ein Endoskop 120 (nur teilweise dargestellt). Das optische System 125 umfasst insbesondere ein Objektiv 126 sowie eine Vielzahl von Lichtleitern 127, etwa Glasfasern, welche ein am Objektiv 126 erfasstes Bild 10 zum Beispiel pixelweise weiterleiten, beispielsweise an ein Photosensor-Array 128. Das Photosensor-Array 128 kann ein Teil des optischen Systems 125 sein, oder zum restlichen Teil des Endoskops 120 gehören. Das optische System 125 kann austauschbar sein, d.h., das Endoskop 120 kann so beschaffen sein, dass das optische System 125 ausgetauscht werden kann, wenn es (zu große) Beeinträchtigungen aufweist. Die Vorrichtung 100 kann ein optisches System 125 oder ein Endoskop 120 umfassen oder separat davon ausgebildet sein. Umfasst die Vorrichtung 100 ein Endoskop 120, kann sie auch als ein Endoskop 120 mit integrierter Zustandserkennung bezeichnet werden.

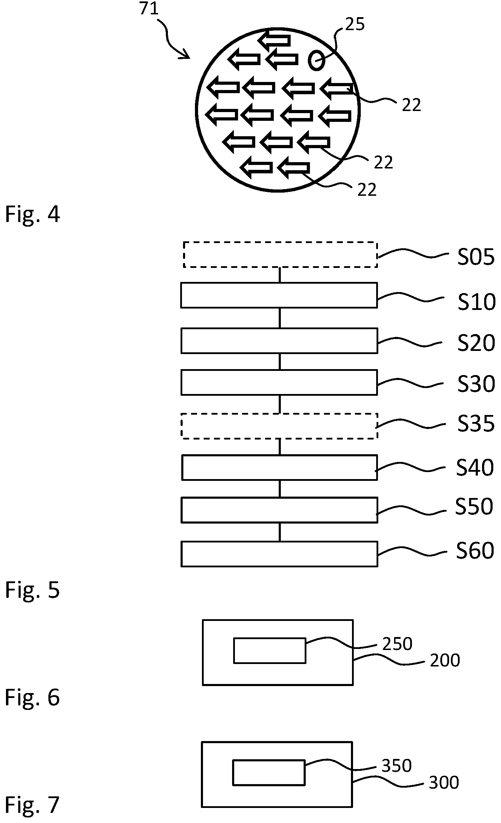

Zurückkehrend zu Fig. 1 ist dort ersichtlich, dass die Vorrichtung 100 eine Eingangsschnittstelle 110 aufweist, welche dazu ausgebildet oder ausgelegt ist, mittels des optischen Systems 125 erfasste Bilder 10, oder, genauer gesagt: darauf basierende digitale Bilddaten 71, zu erhalten. Die Vorrichtung 100 weist außerdem eine Recheneinrichtung 150 auf, welche zumindest ein Auswertemodul 152 implementiert. Die Funktion des Auswertemoduls 152 wird im Folgenden anhand der Figuren 3a, 3b, 3c und 4 näher erläutert werden. Als Beispiel wird für die Erläuterung die Situation gewählt, dass der Zustand des optischen Systems 125 eine Beeinträchtigung des optischen Systems 125 ist, und zwar speziell eine Verschmutzung des optisches Systems 125. Fig. 2 deutet an, dass sich auf dem Objektiv 126 eine solche Verschmutzung 5 befindet, beispielsweise ein angetrockneter Flüssigkeitstropfen oder dergleichen.

Fig. 3a bis Fig. 3c zeigen schematisch von dem Objektiv 126 des optischen Systems 125 erfasste Bilddaten 71, während das Endoskop 120 nach rechts (bezogen auf die Figurenseite) bewegt wird. Das Bild 10 enthält eine eigentlich interessierende Struktur 12, beispielweise menschliches Gewebe eines Patienten oder dergleichen. In den erfassten Bilddaten 71 ist - zusätzlich zu dem eigentlichen Bild 10 mit der interessierenden Struktur 12 - außerdem fälschlich auch ein Bildartefakt 15 zu sehen, welches tatsächlich nicht Teil der erfassten Szenerie ist, sondern ausschließlich auf den Zustand des optischen Systems 125, nämlich die Verschmutzung 5 an dem Objektiv 126, zurückgeht.

Wird das Endoskop 120, genauer gesagt, der Bilderfassungskegel des optischen Systems 125, nach rechts bewegt, wie in den Fig. 3b und Fig. 3c schrittweise dargestellt, verschiebt sich die eigentlich interessierende Struktur 12 in den Bilddaten 71 scheinbar nach links, während das Bildartefakt 15 an seinem Platz bleibt.

Das Auswertemodul 152 ist dazu ausgelegt und eingerichtet, einen gesamten optischen Fluss (oder: optischen Hauptfluss) der erhaltenen Bilddaten 71 zu bestimmen und darauf basierend mindestens einen Bildflussvektor zu identifizieren, insbesondere einen Haupt(bildfluss)vektor oder dominanten optischen Bildflussvektor.

Fig. 4 zeigt schematisch mögliche Bildflussvektoren 22, 25, wie sie das Auswertemodul 152 basierend auf den in den Fig. 3a bis Fig. 3c gezeigten Bilddaten 71 identifizieren könnte. Für das Bestimmen des optischen Flusses können bekannte Verfahren der Videoverarbeitung eingesetzt werden. Typischerweise wird eine Vielzahl von Bildflussvektoren 22, 25 bestimmt werden, beispielsweise für jeden Pixel, für jede in den Bilddaten 71 erkannte Struktur 12, 15, oder dergleichen. In Fig. 4 sind der Übersichtlichkeit halber nur einige wenige Bildflussvektoren 22 eingezeichnet.

Fig. 4 verdeutlicht, dass aufgrund der Bewegung des Endoskops 120 für praktisch alle Bildpunkte oder Pixel der Bilddaten 71 durch das Auswertemodul 152 ein nach links gerichteter Bildflussvektor 22 bestimmt wird, und zwar stets derselbe (oder mit Abweichungen lediglich innerhalb einer Fehlertoleranz oder einer vordefinierten Toleranzschwelle). Wenn das optische System 125 im Ganzen bewegt wird und sonst keine Bewegung in dem Bild 10 stattfindet, stellt die Übereinstimmung aller bestimmten Bildflussvektoren 22 den Normalfall (oder: Soll-Fall) dar. In diesem Fall würde somit auch ein globaler Bildfluss vorliegen bzw. durch das Auswertemodul 152 bestimmbar sein.

Im vorliegenden Fall wird auch noch ein zweiter, von den Bildflussvektoren 22 verschiedener Bildflussvektor 25 bestimmt, welcher auf das Bildartefakt 15 zurückgeht. Dieser wird hier als Nullvektor bestimmt, da sich das Bildartefakt 15 bezüglich des optischen Systems nicht bewegt, und ist in Fig. 4 der Übersichtlichkeit halber mit einer Ellipsenform markiert.

Das Auswertemodul 152 ist nun weiterhin dazu ausgebildet und eingerichtet, falls (wie in Fig. 4) mindestens zwei verschiedene Bildflussvektoren 22, 25 identifiziert wurden (optional: und außerdem das Vorliegen eines globalen Bildflusses festgestellt wurde), basierend darauf mindestens einen Zustand des optischen Systems 125 zu bestimmen.

Hierfür können jedoch Schwellwerte festgelegt sein. Beispielsweise ist es nicht unüblich, dass sich im tatsächlichen Bild 10 etwas bewegt, etwa ein Instrument oder ein Organ. Daher kann vorgesehen sein, dass das Auswertemodul 152 nur dann mindestens einen Zustand des optischen Systems 125 bestimmt, wenn mehr als Y% der ermittelten Bildflussvektoren 22, 25 gleich und nichtverschwindend sind (d.h. ein globaler Bildfluss vorliegt), beispielsweise mehr als 40%, mehr als 50%, mehr als 60%, mehr als 70%, mehr als 80%, mehr als 90%, mehr als 90%, mehr als 95%, oder mehr als 98% betragen.

In diesem Fall kann nämlich darauf geschlossen werden, dass sich nicht einzelne Objekte im Bild 10 bewegen, sondern dass ein Schwenk bzw. eine Bewegung des Endoskops 120 (oder zumindest des optischen Systems 125) stattgefunden hat, d.h. eine großflächige Bildänderung, und somit eine Mehrzahl verschiedener Bildflussvektoren 22, 25 tatsächlich einen beeinträchtigten Zustand des optischen Systems 125 indiziert, d.h., anzeigt.

In verschiedenen Varianten kann entweder vorgesehen sein, dass ein Zustand des optischen Systems 125 durch das Auswertemodul 152 nur dann bestimmt wird, wenn ein globaler Bildfluss vorliegt, oder alternativ vorgesehen sein, der Zustand immer bestimmt wird, oder immer, wenn mehr als zwei Bildflussvektoren vorliegen, aber das Vorliegen eines globalen Bildflusses beim Bestimmen des Zustands berücksichtigt wird.

Zusätzlich oder alternativ kann auch vorgesehen sein, dass das Bestimmen der Bildflussvektoren 22, 25 über einen vorbestimmten oder einstellbaren Mindestzeitraum hinweg erfolgt.

Zum konkreten Bestimmen des Zustands des optischen Systems 125 kann das Auswertemodul 152 verschiedene Verfahren verwenden. Die Information über das Vorliegen z.B. von Nullvektoren in einem globalen Bildfluss ist ein Hinweis auf nicht-funktionale (oder: nicht zuverlässige) Pixel. Die Form oder Anordnung solcher Pixel oder Cluster von Pixel kann bereits einen Hinweis auf die Art der Beeinträchtigung geben. Somit kann das Auswertemodul 152 mit Regeln ausgestattet sein, die aufgrund der geometrischen Form oder Anordnung von Pixeln oder Clustern von Pixeln mit Nullvektoren im globalen Bildfluss auf die Art der Beeinträchtigung schließen. Beispielsweise kann ein länglicher Cluster auf einen Kratzer im Objektiv 126 hinweisen, ein runder oder elliptischer Cluster auf einen Fleck auf dem Objektiv 126 hinweisen, und ein runder und scharfer Cluster auf einen Einschlag in dem Objektiv 126 hinweisen. Ein einzelnes nicht-funktionales Pixel kann auf eine gebrochene Litze unter den Lichtleitern 127 hinweisen.

Die Auswertung von Form und/oder Anordnung von nicht-funktionalen Pixeln oder Clustern von nicht-funktionalen Pixeln kann durch eine inhaltliche und/oder quantitative Bildauswertung ergänzt werden. Beispielsweise ist es bei einer gebrochenen Litze oft der Fall, dass das nicht-funktionale Pixel von einem "Hof" von eingeschränktfunktionalen Pixeln umgeben ist, was durch eine quantitative Bildauswertung ermittelt werden und beim Bestimmen des Zustands berücksichtigt werden kann.

Mehrere Bildflussvektoren 22, 25 unterschiedlicher Größe und Richtung könnten auf eine Rauchgasausbreitung oder Zug hinweisen und somit keinen beeinträchtigten Zustand des optischen Systems 125 darstellen.

Alternativ oder zusätzlich kann eine Bestimmung der Art der Beeinträchtigung, als Teil des Bestimmens des Zustand des optischen Systems 125, auch mittels einer Bilddifferenzbildung erfolgen. Hierzu kann der Unterschied zwischen den jüngsten Bilddaten 71 und älteren Bilddaten 71 aus einer Bildstromsequenz von Bilddaten 71, oder zwischen den jüngsten Bilddaten 71 und einem virtuellen Hintergrund ermittelt werden. Der virtuelle Hintergrund kann beispielsweise durch eine Mittelung der N letzten Bilddaten 71 aus einer Bildstromsequenz erzeugt werden.

Somit kann das Auswertemodul 152 die Art der Beeinträchtigung auch mittels einer Hintergrunddifferenzanalyse und/oder einer Vorbildsequenzdifferenzanalyse bestimmen. Beide Techniken sind an sich im Stand der Technik bekannt. Insbesondere können so Position und Größe von Defekten des optischen Systems 125, insbesondere des Objektivs 126 und/oder der Lichtleiter 127, bestimmt werden.

Je nach konkreter Anwendung können die bestimmbaren Zustände unterschiedlich definiert werden. Beispielsweise kann bei einer ersten Variante ein Zustand lauten: "Defekt in den Pixeln A-B, Verschmutzung der Pixel C-D, Bruch der Litzen E-F", während bei einer zweiten Variante dieselbe Situation durch drei verschiedene Zustände 1)-3) ausgedrückt werden kann:
Zustand 1): Defekt in den Pixeln A-B
Zustand 2): Verschmutzung der Pixel C-D
Zustand 3): Bruch der Litzen E-F.

Die Vorrichtung 100 verfügt außerdem über eine Ausgabeschnittstelle 190, welche dazu eingerichtet ist, ein Ausgabesignal 79 zu erzeugen, welches zumindest eine Information über den bestimmten mindestens einen Zustand des optischen Systems 125 aufweist. In einem simplen Fall kann das Ausgabesignal 79 lediglich indizieren, dass eine Beeinträchtigung vorliegt, sodass ein Benutzer das optische System 125 bei nächster Gelegenheit inspizieren kann. Alternativ kann eine der im Vorangehenden beschriebenen Varianten oder Alternativen vorgesehen sein, wonach die Ausgabeschnittstelle 190 eine Handlungsaufforderung und/oder ein Steuersignal zum automatischen Beheben oder verbessern des mindestens einen bestimmten Zustands des optischen Systems 125 ausgeben kann, entweder als das Ausgabesignal 79, als Teil des Ausgabesignals 79, oder zusätzlich dazu. Bei einem Zustand mit mehreren Beeinträchtigungen, oder mehreren bestimmten Zuständen mit jeweils unterschiedlichen Beeinträchtigungen, kann das Ausgabesignal 79 selbstverständlich entsprechend angepasst werden, für jede Beeinträchtigung eine Information, eine Handlungsaufforderung, eine Steuersignal etc. zu umfassen.

Im vorliegend anhand der Fig. 2-4 beschriebenen Fall könnte beispielsweise eine Anzeigeeinrichtung (etwa ein Display oder ein Touchscreen) gesteuert werden, außer den von dem optischen System 125 erfassten Bilddaten 71 zusätzlich noch den Hinweis "Objektiv reinigen!" einzublenden, oder dergleichen. Alternativ könnte eine akustische Ausgabeeinrichtung (etwa ein Lautsprecher oder ein Kopfhörer) gesteuert werden, ein akustisches Warnsignal, z.B. einen Warnton oder eine Sprachausgabe "Warnung: Objektiv reinigen" oder dergleichen auszugeben.

Wird, wie im Vorangehenden ebenfalls erläutert wurde, eine verbleibende Nutzungsdauer (oder, äquivalent, ein Datum für das geschätzte Nutzungsdauerende, oder eine Anzahl verbleibender Verwendungen des optischen Systems 125) bestimmt, kann durch die Anzeigeeinrichtung auch diese angezeigt werden, insbesondere auch dauerhaft und/oder regelmäßig und/oder dynamisch aktualisiert.

Die Bildverarbeitung der von dem optischen System 125 erfassten Bilddaten 71 erfolgt üblicherweise in einem Gerät, das als "Kamerasteuerung" bezeichnet wird. Dementsprechend ist es vorteilhaft, wenn die erfindungsgemäße Vorrichtung 100 eine solche Kamerasteuerung umfasst oder in eine solche Kamerasteuerung integriert ist. Teile des Auswertemoduls 152 können dann beispielsweise durch in der Kamerasteuerung ohnehin ablaufende Prozesse der Videoverarbeitung durchgeführt werden. Darüber hinaus ist die Kamerasteuerung üblicherweise dazu eingerichtet, eine Anzeigeeinrichtung zum Anzeigen der erfassten Bilddaten 71 zu steuern und umfasst somit ebenfalls eine Ausgabeschnittstelle, welche die Aufgaben auch der Ausgabeschnittstelle 190 der erfindungsgemäßen Vorrichtung 100 ausführen kann. Die Vorrichtung 100 kann aber auch ganz oder teilweise räumlich entfernt (engl. "remote") implementiert sein, etwa durch eine Cloud-Computing-Plattform.

Die Vorrichtung 100 kann im Rahmen eines Instrumenten-Managementsystems die Ausgabesignale 79 für eine Vielzahl von optischen Systemen 125 sammeln. Hierzu kann die Vorrichtung 100 beispielsweise ein Instrumenten-Management-Modul 154 aufweisen. Jedes optische System 125 weist bevorzugt eine individuelle Kennung (z.B. einen alphanumerischen Kenncode) auf, durch welche der jeweilige bestimmte Zustand eineindeutig dem jeweiligen optischen System 125 zuordenbar ist. Durch Instrumenten-Management-Modul 154 können somit beispielsweise, wie im Vorangehenden bereits beschrieben wurde, von dem Auswertemodul 152 bestimmte quantitative Einstufungen jedes bestimmten Zustands jedes optischen Systems 125 erfasst und gespeichert werden, optional zusammen mit einer bestimmten Geschwindigkeit der Veränderung des Zustands oder der quantitativen Einstufung und/oder mit einer (beispielsweise auf der Geschwindigkeit der Veränderung basiert geschätzten) verbleibenden Nutzungsdauer des jeweiligen optischen Systems 125. Die Informationen über die bestimmten Zustände können in einem maschinenlesbaren Bericht mit vordefinierten Feldern ausgegeben und/oder gespeichert werden.

Dies ermöglicht eine sehr präzise und effektive vorausschauende Wartung, da für jedes optische System 125 jederzeit bekannt ist, ob und wie starke Beeinträchtigungen es aufweist und wann diese - gegebenenfalls - so große Ausmaße angenommen haben (werden), dass das entsprechende optische System 125 ausgetauscht werden muss. Das Instrumenten-Management-Modul 154 kann so eingerichtet sein, dass der entsprechende Austausch oder sogar die Produktion eines Ersatzteils automatisch beauftragt oder gesteuert wird.

Weist die erfindungsgemäße Vorrichtung 100 ein Instrumenten-Management-Modul 154 auf, ist es bevorzugt, dass zumindest das Instrumenten-Management-Modul 154, oder die gesamte Vorrichtung 100, zentral implementiert ist, etwa durch einen Server einer medizinischen Institution (Krankenhaus, Forschungseinrichtung, Arztpraxis...), durch eine Cloud-Computing-Plattform oder dergleichen, damit eine Vielzahl von Bilddaten 71 einer Vielzahl von verschiedenen optischen Systemen 125 in einfacher Weise zu der Vorrichtung 100 gelangen und von dieser ausgewertet werden kann. Fig. 5 zeigt ein schematisches Flussdiagramm zum Erläutern eines Verfahrens gemäß einer weiteren Ausführungsform der vorliegenden Erfindung. Das erfindungsgemäße Verfahren ist insbesondere mittels der erfindungsgemäßen Vorrichtung 100 ausführbar, aber auch separat davon. Dementsprechend ist das erfindungsgemäße Verfahren gemäß allen in Bezug auf die erfindungsgemäße Vorrichtung, insbesondere im Vorangehenden mit Bezug auf die Figuren 1-4, beschriebenen Optionen, Varianten, Weiterbildungen und Verfeinerungen anpassbar und umgekehrt.

In einem Schritt S10 werden Bilddaten 71 erhalten, welche durch das optische System 125 erfasst wurden. Dies kann beispielsweise geschehen, wie im Vorangehenden bereits mit Bezug auf die Eingangsschnittstelle 110 beschrieben wurde. Das Verfahren kann optional auch einen vorangehenden Schritt S05 des Erfassens von Bilddaten 71 durch das optische System 125 umfassen.

In einem Schritt S20 wird ein gesamter optischer Fluss (oder: optischer Hauptfluss) in den erhaltenen Bilddaten 71 bestimmt, insbesondere wie im Vorangehenden mit Bezug auf das Auswertemodul 152 und die Fig. 3a bis 4 beschrieben wurde. Beispielsweise kann hierzu eine typische Videoverarbeitung ausgeführt werden.

In einem Schritt S30 wird in dem bestimmten optischen Fluss mindestens ein Bildflussvektor 22, 25 identifiziert, insbesondere wie im Vorangehenden mit Bezug auf das Auswertemodul 152 und Fig. 4 beschrieben wurde. Bei dem mindestens einen bestimmen (oder: zu bestimmenden) Bildflussvektor 22, 25 kann es sich insbesondere um einen Haupt(bildfluss)vektor oder dominanten optischen Bildflussvektor handeln.

In einem optionalen Schritt S35 wird bestimmt, ob ein globaler Bildfluss vorliegt, wie im Vorangehenden ebenfalls bereits ausführlich erläutert wurde. Das Vorliegen eines globalen Bildflusses kann als optional zusätzlich notwendiges Kriterium dafür verwendet werden, dass ein Zustand des optischen Systems 125 bestimmt wird, oder dass ein beeinträchtigter Zustand des optischen Systems 125 bestimmbar ist, oder dergleichen mehr.

Falls in dem Schritt S30 mindestens zwei verschiedene Bildflussvektoren 22, 25 identifiziert wurden (optional: und zusätzlich in dem Schritt S35 das Vorliegen eines globalen Bildflusses bestimmt wurde), wird in einem Schritt S40 basierend auf den mindestens 2 verschiedenen identifizierten Bildflussvektoren 22, 25 mindestens ein Zustand des optischen Systems 125 bestimmt, beispielsweise wie im Vorangehenden mit Bezug auf das Auswertemodul 152 oder allgemein mit Bezug auf die Erfindung erläutert wurde.

In einem Schritt S50 kann basierend auf dem mindestens einen bestimmten Zustand ein Ausgabesignal 79 ausgegeben werden, beispielsweise wie im Vorangehenden mit Bezug auf die Ausgabeschnittstelle 190 erläutert wurde. Das Ausgabesignal 79 enthält zumindest eine Information über den mindestens einen bestimmten Zustand, optional auch über eine quantitative Einstufung des mindestens einen bestimmten Zustands, und kann insbesondere eine Handlungsaufforderung und/oder ein Steuersignal aufweisen.

In einem Schritt S60 kann mittels eines solchen Steuersignals eine Hinweiseinrichtung (z.B. eine Anzeigeeinrichtung oder eine akustische Ausgabeeinrichtung) gesteuert werden, einen Benutzer über den mindestens einen bestimmten Zustand des optischen Systems 125 zu informieren, ihm eine Handlungsanweisung (z.B. "Objektiv reinigen!") visuell anzuzeigen und/oder akustisch mitzuteilen.

Fig. 6 zeigt ein schematisches Blockdiagramm eines Computerprogrammprodukts 200 gemäß einer Ausführungsform des dritten Aspekts der vorliegenden Erfindung. Das Computerprogrammprodukt 200 umfasst ausführbaren Programmcode 250, welcher dazu eingerichtet ist, wenn er ausgeführt wird (z.B. durch eine Recheneinrichtung), das Verfahren gemäß einer Ausführungsform der vorliegenden Erfindung durchzuführen, beispielsweise gemäß Fig. 3 bis Fig. 5.

Fig. 7 zeigt ein schematisches Blockdiagramm eines nichtflüchtigen computerlesbaren Datenspeichermediums 300 gemäß einer Ausführungsform der vorliegenden Erfindung. Das Datenspeichermedium 300 umfasst ausführbaren Programmcode 350, welcher dazu eingerichtet ist, wenn er ausgeführt wird (z.B. durch eine Recheneinrichtung), das Verfahren gemäß einer Ausführungsform der vorliegenden Erfindung durchzuführen, beispielsweise gemäß Fig. 3 bis Fig. 5.

Das nicht-flüchtige computerlesbare Datenspeichermedium 300 kann beispielsweise als ein Halbleiterspeicher, z. B. ein SSD-Speicherstein ausgebildet sein oder einen solchen aufweisen. Das Datenspeichermedium 300 kann auch eine CD, DVD, Blu-Ray oder eine magnetische Speichervorrichtung aufweisen oder umfassen.

Die vorstehende Beschreibung der offenbarten Ausführungsformen enthält lediglich Beispiele für mögliche Umsetzungen, die beschrieben werden, um einen Fachmann in die Lage zu versetzen, die vorliegende Erfindung herzustellen oder verwenden zu können. Verschiedene Variationen und Modifikationen dieser Ausführungsformen sind für den Fachmann - nach Kenntnis der vorliegenden Erfindung - leicht ersichtlich, und die hierin definierten allgemeinen Prinzipien können auf andere Ausführungsformen angewendet werden, ohne vom Umfang der vorliegenden Offenbarung abzuweichen.

Somit soll die vorliegende Erfindung nicht auf die hierin gezeigten, spezifischen Ausführungsformen beschränkt sein, sondern ihr soll der breiteste Umfang zugestanden werden, der mit den hierin offenbarten Prinzipien und neuen Merkmalen übereinstimmt. Daher ist die vorliegende Erfindung nur in Übereinstimmung mit den folgenden Ansprüchen einzuschränken.

Die Erfindung lässt sich wie folgt grob zusammenfassen: in Bilddaten 71 eines optischen Systems 125 eines Endoskops 120 werden Bildflussvektoren 22, 25 bestimmt und darauf basierend ermittelt, ob das Endoskop 120 Beeinträchtigungen, z. B. Defekte oder Verschmutzungen, aufweist. Dies erfolgt bevorzugt nur dann, wenn ein globaler Bildfluss festgestellt wurde, und darin einzelne Bildflussvektoren mit dem festgestellten globalen Bildfluss nichtkonform sind.

### Bezugszeichenliste

- 5: Verschmutzung
- 10: Bild
- 12: interessierende Struktur
- 15: Bildartefakt
- 22: Bildflussvektor
- 25: Bildfluss(null-)vektor
- 71: digitale Bilddaten
- 79: Ausgabesignal
- 100: Vorrichtung
- 110: Eingangsschnittstelle
- 120: Endoskop
- 125: optisches System
- 126: Objektiv
- 127: Lichtleiter
- 128: Photosensor-Array
- 150: Recheneinrichtung
- 152: Auswertemodul
- 154: Instrumenten-Management-Modul
- 190: Ausgabeschnittstelle
- 200: Computerprogrammprodukt
- 250: Programmcode
- 300: Datenspeichermedium
- 350: Programmcode
- S05..S60: Verfahrensschritte

## Patentansprüche

1. Vorrichtung (100) zum Bestimmen eines Zustands eines optischen Systems (125) für ein Endoskop (120), umfassend:
eine Eingangsschnittstelle (110), welche dazu eingerichtet ist, mittels des optischen Systems (125) erfasste Bilddaten (71) zu erhalten;
einer Recheneinrichtung (150), welche dazu eingerichtet ist, zumindest ein Auswertemodul (152) zu implementieren, welches dazu eingerichtet ist, einen gesamten optischen Fluss der erhaltenen Bilddaten (71) zu bestimmen (S20), darauf basierend mindestens einen Bildflussvektor (22, 25) zu identifizieren (S30), und, falls mindestens zwei verschiedene Bildflussvektoren (22, 25) identifiziert wurden, basierend darauf mindestens einen Zustand des optischen Systems (125) zu bestimmen (S40); und
einer Ausgabeschnittstelle (190), welche dazu eingerichtet ist, ein Ausgabesignal (79) zu erzeugen, welches eine Information über den bestimmten mindestens einen Zustand des optischen Systems (125) aufweist.

2. Vorrichtung (100) nach Anspruch 1,
wobei das Auswertemodul (152) dazu eingerichtet ist, anhand der mindestens zwei identifizierten verschiedenen Bildflussvektoren (22, 25) mindestens einen der folgenden Zustände des optischen Systems (125) zu bestimmen:
- eine Verschmutzung des optischen Systems (125);
- eine Beschädigung des optischen Systems (125);
- eine verbleibende Nutzungsdauer des optischen Systems (125); und/oder
- einen Funktionalitätsgrad des optischen Systems (125).

3. Vorrichtung (100) nach Anspruch 1 oder 2,
wobei die Ausgabeschnittstelle (190) außerdem dazu eingerichtet ist, eine Handlungsaufforderung an einen Benutzer zum Beheben oder Verbessern des mindestens einen bestimmten Zustands des optischen Systems (125) auszugeben und/oder ein Steuersignal zum automatischen Beheben oder Verbessern des mindestens einen bestimmten Zustands des optischen Systems (125) auszugeben.

4. Vorrichtung (100) nach einem der Ansprüche 1 bis 3,
wobei das Auswertemodul (152) außerdem dazu eingerichtet ist, eine Geschwindigkeit einer Veränderung mindestens eines bestimmten Zustands des optischen Systems (125) zu bestimmen.

5. Vorrichtung (100) nach Anspruch 4,
wobei das Auswertemodul (152) außerdem dazu eingerichtet ist, basierend auf der bestimmen Geschwindigkeit der Veränderung des mindestens einen bestimmten Zustands des optischen Systems (125) eine vorausschauende Wartung zu implementieren.

6. Vorrichtung (100) nach einem der Ansprüche 1 bis 5, wobei das Auswertemodul (152) dazu eingerichtet ist, das Bestimmen (S20) des optischen Flusses, das Identifizieren (S30) des mindestens einen Bildflussvektors (22, 25) und/oder das Bestimmen (S40) des mindestens einen Zustands des optischen Systems (125) in einem Normalbetrieb zur Laufzeit des optischen Systems (125) durchzuführen.

7. Vorrichtung (100) nach einem der Ansprüche 1 bis 5, wobei das Auswertemodul (152) dazu eingerichtet ist, das Bestimmen (S20) des optischen Flusses, das Identifizieren (S30) des mindestens einen Bildflussvektors (22, 25) und/oder das Bestimmen (S40) des mindestens einen Zustands des optischen Systems (125) ausschließlich in einem gesonderten Testbetrieb des optischen Systems (125) durchzuführen, wobei einem Benutzer optional durch das Auswertemodul (152) Handlungsanweisungen zum Durchführen des Testbetriebs bereitgestellt werden.

8. Computerimplementiertes Verfahren zum Bestimmen eines Zustands eines optischen Systems (125) für ein Endoskop (120), umfassend:
Erhalten (S10) von Bilddaten (71), welche durch das optische System (125) erfasst wurden;
Bestimmen (S20) eines gesamten optischen Flusses in den erhaltenen Bilddaten (71);
Identifizieren (S30) mindestens eines Bildflussvektors (22, 25) in dem bestimmten optischen Fluss; und,
falls mindestens zwei verschiedene Bildflussvektoren (22, 25) identifiziert wurden:
Bestimmen (S40), basierend auf den mindestens zwei verschiedenen identifizierten Bildflussvektoren (22, 25), mindestens eines Zustands des optischen Systems (125).

9. Computerprogrammprodukt (200), umfassend ausführbaren Programmcode (250), welcher dazu ausgelegt ist, wenn er durch eine Recheneinrichtung ausgeführt wird, das Verfahren gemäß Anspruch 8 auszuführen.

10. Computerlesbares, nicht-flüchtiges Datenspeichermedium (300), umfassend ausführbaren Programmcode (350), welcher dazu ausgelegt ist, wenn er durch eine Recheneinrichtung ausgeführt wird, das Verfahren gemäß Anspruch 8 auszuführen.
